# EUROPEAN PATENT SPECIFICATION

(11) **EP 0 710 224 B1**
(45) Date of publication and mention of the grant of the patent: **15.04.1998**
(21) Application number: 94924787.8
(22) Date of filing: 15.07.1994
(51) Int. Cl.: C07C 67/38, C07C 69/54, C07C 69/533, C07C 69/618

(54) **PROCESS FOR THE CARBONYLATION OF ACETYLENICALLY UNSATURATED COMPOUNDS**
VERFAHREN ZUR CARBONYLIERUNG VON ACETYLENISCH UNGESÄTTIGTEN VERBINDUNGEN
PROCEDE DE CARBONYLATION DE COMPOSES ACETYLENIQUEMENT INSATURES

(30) Priority: 19.07.1993 EP 93202113
(43) Date of publication of application: 08.05.1996
(73) Proprietor: SHELL INTERNATIONALE RESEARCH MAATSCHAPPIJ B.V., 2596 HR Den Haag (NL)
(72) Inventor: DRENT, Eit, NL-1031 CM Amsterdam (NL); PELLO, Dennis, Humphrey, Louis, NL-1031 CM Amsterdam (NL)
(74) Representative: Zeestraten, Albertus Wilhelmus Joannes
(86) International application number: EP9402386
(87) International publication number: WO9503269

(56) References cited:
- EP-A- 0 186 228
- EP-A- 0 495 547

## Description

The invention relates to a process for the preparation of carbonylated products by reacting acetylenically unsaturated compounds with carbon monoxide in the presence of a co-reactant and a palladium containing catalyst.

EP-A-495547 relates to a process for the monocarbonylation of optionally substituted olefinically unsaturated compounds. Such process differs fundamentally from the present process in the kind of feedstock employed.

A process of the type of the present invention is disclosed in EP-186228. According to this document acetylenically unsaturated compounds are carbonylated with carbon monoxide in the presence of an alcohol and/or water and of a liquid phase, using a catalyst system formed by combining a compound of divalent palladium, an organic phosphine and a protonic acid, other than hydrohalogenic acids. Whereas the definition of the phosphorus compound broadly covers all categories of organic phosphines, the known process is specifically illustrated by experiments, carried out in the presence of a monophosphine. In two experiments a catalyst system comprising a mixture of a monophosphine and a minor amount of a diphosphine containing 4 aryl groups is used, but the obtained reaction rates in these examples are low.

It is emphasized in EP-186228 that high reaction rates are in particular obtained by using a catalyst system comprising a phosphine of the general formula PQ¹Q²Q³ in which Q¹,Q² and Q³ each represent a phenylgroup, preferably carrying an electron withdrawing substituent. Moreover, it is stated that high reaction rates and selectivities are obtained when more than 5 and in particular more than 20 moles of organic phosphine per mole of palladium are present. Usually, reaction temperatures of 115 °C are applied.

Unexpectedly, it has now been found that by using a catalyst system, based, as regards the phosphorus compound, on specific bidentate diphosphines, not only lower amounts of phosphine suffice, but also that at significantly lower temperatures high reaction rates and high selectivities are achieved. Furthermore, the selectivity with respect to linear or non-linear carbonylated products can be increased, depending on the acid component on which the catalyst system is based.

The invention may be defined as relating to a process for the carbonylation of acetylenically unsaturated compounds with carbon monoxide in the presence of a nucleophilic compound having one or more mobile hydrogen atoms as co-reactant, and of a catalyst system based on
(a) a source of palladium cations;
(b) a bidentate diphosphine of the general formula

   R¹R²P-R-PR³R⁴ (I)

   wherein each of R¹,R²,R³ and R⁴ independently represents a substituted or non-substituted aliphatic or cycloaliphatic group, or R¹ together with R², and/or R³ together with R⁴ represent a substituted or non-substituted bivalent cyclic group with at least 5 ring atoms whereby the two free valencies of such a cyclic group are linked to a single phosphorus atom, and R represents a bivalent organic bridging group containing from 1 to 4 atoms in the bridge, and
(c) a source of non- or weakly coordinating anions derivable from strong acids having a pKa not greater than 4, or a source of anions derivable from sterically hindered carboxylic acids.

It is considered likely that the reaction according to the invention involves the formation of an intermediate complex between a palladium cation- in coordination with two phosphorus atoms of the bidentate diphosphine- and an acetylenically unsaturated compound.

It is believed that in this complex palladium is initially linked to the unsaturated moiety of the acetylenically unsaturated compound and that subsequently carbon monoxide reacts with the complex.

The source of palladium cations is advantageously a palladium salt. Suitable salts are, for example, salts derived from nitric acid, sulphuric acid or a sulphonic acid, such as p-toluenesulphonic acid, methanesulphonic acid or trifluoromethanesulphonic acid.

Preferably, a palladium salt of a carboxylic acid is used, in particular of a carboxylic acid having not more than 12 carbon atoms. Examples of suitable carboxylic acids are acetic acid, chloroacetic acid, trifluoroacetic acid, propionic acid and hexanoic acid. Palladium acetate is a particular preferred source of palladium cations.

If desired, metallic palladium may be used as well, or a palladium complex, such as paliadium acetylacetonate, tetrakis (triphenylphosphine) palladium or bis (triphenylphosphine) palladium sulphate. A heterogeneous source of palladium cations may also be used, for example palladium bound to a sulphonated ion-exchange resin.

In the bidentate diphosphines of the above formula (I), R¹,R²,R³ and R⁴ may independently represent any substituted or non-substituted aliphatic or cycloaliphatic group.

Suitable aliphatic groups include alkyl groups having from 1 to 10 carbon atoms, in particular from 1 to 6 carbon atoms. Most preferred are alkyl groups containing from 2 to 5 carbon atoms. The alkyl groups may be linear or branched. When R¹,R²,R³ and/or R⁴ represents a substituted alkylgroup, any such substituted alkylgroup preferably contains not more than one substituent.

Examples of suitable alkylgroups include methyl, ethyl, propyl, butyl and pentyl groups. Examples of preferred alkylgroups are isopropyl, secondary butyl and tertiary butyl groups. As an especially preferred bidentate diphosphine the compound 1,3- bis (di-secondary butylphosphino) propane may be mentioned.

Suitable cycloaliphatic groups include cyclic structures with up to 12 ring atoms, preferably cycloalkyl groups with 5 to 8 ring atoms, such as cyclohexyl- and cyclooctylgroups.

The bivalent cyclic groups that may be represented by R¹ together with R², and/or R³ together with R⁴, comprise at least 5 ring atoms, preferably from 6 to 10 ring atoms, in particular from 7 to 9 ring atoms. As a rule they comprise only carbon atoms as ring atoms, but cyclic groups containing one or two hetero- atoms in the ring, for example an oxygen atom, are not precluded.

The two free valencies which are linked to a single phosphorus atom, may occur at adjacent carbon ring atoms, or at two carbon ring atoms which are further apart. Examples of suitable bivalent cyclic groups are 1,2-cyclooctylene, 1,3-cyclo octylene, 1,4 cyclooctylene, 1,5-cyclooctylene, 1,4-cyclohexylene and 1,4-cycloheptylene.

Bidentate diphosphines of formula (I) containing two identical bivalent cyclic groups are preferred, mainly in view of ease of preparation. In particular preferred bidentate diphosphines are [3.3.1] and/or [4.2.1] isomers of 1,2 -bis(9-phosphabicyclononyl)ethane, 1,2-bis(9-phosphabicyclononyl)propane and 1,3-bis(9-phosphabicyclononyl)propane or mixtures thereof. For the preparation of these compounds and the like, known techniques are available, e.g. the method disclosed in UK-1,127,965.

In the bidentate diphosphines of formula (I) the bridging group R is preferably selected such that the two phosphorus atoms to which R is linked, can easily coordinate with the palladium cation. Preferably R represents a bridging group containing from 1 to 4 carbon atoms in the bridge. A non terminal carbon atom in the bridge may carry a non-bulky substituent, if so desired, but in general unsubstituted bridging groups are preferred. Bidentate diphosphines wherein R represents an ethylene or a trimethylene group are in particular preferred.

Any substituents present in one or more of the groups represented by R¹,R²,R³,R⁴ and R, are preferably selected from the group consisting of halogen atoms, cyano, alkoxy, acyl, alkylamino and dialkylamino groups. The alkylgroup(s) in any alkoxy, acyl, alkylamino or dialkylaminogroup preferably contain(s) from 1 to 4 carbon atoms.

The source of anions on which the catalyst system is based (component c)), can be any compound which is capable of generating anions such as acids and metal salts, for example salts of vanadium, chromium, nickel, copper or silver. Advantageously a palladium salt may be used, acting both as a source of anions and as a source of palladium cations.

It has been observed that the formation of linear carbonylated products is considerably enhanced, if the catalyst system is based on a source of non- or weakly- coordinating anions, i.e. on a source of anions which have no, or only to a minor extent covalent interaction with the palladium cation.

Non- or weakly-coordinating anions are preferably derived from strong acids having a pKa not greater than 4 (measured in aqueous solution at 18 °C), or salts of such acids. Preferably acids are selected having a pKa not greater tnan 2. Typical examples of non- or weakly coordinating anion sources are nitric acid, sulphuric acid, sulphonic acids, such as chlorosulphonic acid, fluorosulphonic acid, and trifluoromethanesulphonic acid, hydrocarbylsulphonic acids such as benzenesulphonic acid, methanesulphonic acid and tert-butylsulphonic acid and phosphonic acids.

A preferred source of non- or weakly coordinating anions is methanes phonic acid.

Furthermore sources of anions derived from sterically hindered carboxylic acids may be used, in particular if the formation of non-linear carbonylated products is envisaged. Such carboxylic acids comprise one or more bulky groups in the vicinity of the carboxyl group.

Examples of suitable sterically hindered carboxylic acids are phenylphosphoric acid, benzenephosphonic acid, 2,5-dihydroxybenzoic acid, 2,6-dihydroxybenzoic acid, 1-naphthylcarboxylic acid and 9-anthracenylcarboxylic acid. The sterically hindered 9-anthracenylcarboxylic acid is in particular preferred.

The number of moles of bidentate diphosphines of formula (I) per mole of palladium may vary and is usually in the range of 0.5 to 5.

It has been found that supplying higher amounts of diphosphines does not result in significantly higher reaction rates or selectivities and is moreover unattractive for economic reasons.

Preferably, the amount of diphosphine is selected in the range of 0.5 to 3 moles of diphosphine per mole of palladium.

Conveniently, the catalyst is applied in an amount such that per mole of acetylenically unsaturated compound 10⁻⁷ to 10⁻¹ mole of palladium is present, preferably 10⁻⁶ to 10⁻¹ mole of palladium on the same basis.

The acetylenically unsaturated compounds, suitable to be used in the process of the invention, include acetylenically unsaturated compounds containing from 2 to 20 carbon atoms, optionally comprising one or more inert substituents. Preferably unsubstituted hydrocarbons containing a single acetylenically unsaturated bond are used, which bond may be terminal or internal.

Very suitable are compounds having from 2 to 9 carbon atoms. Typical examples of recommended starting materials are ethyne, propyne, 1-butyne, 2 butyne, 1-pentyne, phenylethyne and para-tolueneethyne.

The carbonylated products obtained with ethyne, propyne and phenylethyne have established industrial uses and, accordingly, the process of the invention is of particular interest for the conversion of these starting materials.

As co-reactant any nucleophilic compound having one or more mobile hydrogen atoms may be used. Suitable nucleophilic compounds include alcohols, phenols, thiols, water and amines. Preferably, a co reactant is selected from the group consisting of alcohols, phenols and thiols.

Examples of suitable co-reactants are monohydric alcohols such as methanol, ethanol, isopropanol, 1-butanol, 2-butanol, tert-butanol, n-hexanol, n-octanol and n-decanol, polyhydric alcohols such as ethyleneglycol, 1,3-propanediol, 1,4 butanediol and pentaerythritol, phenols such as phenol, naphthol, catechol and hydroquinone and thiols such as 1-mercaptopropane and 1-mercaptobutane.

Alcohols having from 1 to 6 carbon atoms are preferred nucleophilic co-reactants, in particular methanol and 1-butanol.

If desired, the process may be carried out in the additional presence of an inert diluent. When a diluent is employed preferably aprotic solvents are used such as ethers, for example diethylether, 2,5,8-trioxanonane (diglyme) and tetrahydrofuran, hydrocarbons such as n-hexane and toluene and ketones such as acetone and methylisobutylketone.

Ethers, in particular 2,5,8-trioxanonane, are preferred solvents.

The process of the invention is carried out at moderate reaction conditions. It has been bserved that at relatively low reaction temperatures, e.g. at temperatures below 100 °C and often even below 90 °C, high conversion rates and high selectivities with respect to the desired reaction product are achieved.

In general, the reaction temperature will be in the range of 50 to 140 °C, preferably in the range of 60 to 100 °C.

Preferably, the total reaction pressure is in the range of 2 to 80 bar, pressures outside this range not being precluded.

The process may be carried out batchwise, or in continuous operation.

The invention is illustrated by the following examples.

### Examples I-V

The experiments were carried out by each time charaing a 250 mL magnetically stirred Hastelloy C (Trade Mark) autoclave with the acetylenically unsaturated compound, the co-reactant (methanol) and, whenever employed, the solvent.

The autoclave was pressurized with carbon monoxide at 40 bar and the catalyst solution was injected under carbon monoxide pressure. The catalyst solution comprised 0.1 mmol of palladium (II) acetate, the bidentate diphosphine and acid, dissolved in methanol.

Subsequently, the carbon monoxide pressure was brought at the desired value and the contents of the autoclave were heated to the set reaction temperature.

The catalyst components (other than palladium acetate), the acetylenically unsaturated compound, the co-reactant and, if present, the solvent, as well as the pressure and temperature values, are compiled in Table 1.

After the reaction, the mixture was cooled and the pressure released. The products, mainly linear and non-linear unsaturated esters, were analysed by means of Gas Liquid Chromatography.

The results, as regards reaction rate and selectivity with respect to linear and non-linear product are shown in Table 1.

The abbreviations used therein, have the following meaning;
- BPBNE =: 1,2-bis(9-phospha-bicyclononyl)ethane
- BDTPP =: 1,3-bis (di-tert-butylphosphino)propane
- BPBNP =: 1,3-bis(9-phospha-bicyclononyl)propane
- BDSPP =: 1,3-bis(di-sec-butylphosphino)propane
- MS =: methanesulphonic acid
- TMS =: trifluoromethanesulphonic acid
- TA =: trifluoroacetic acid.

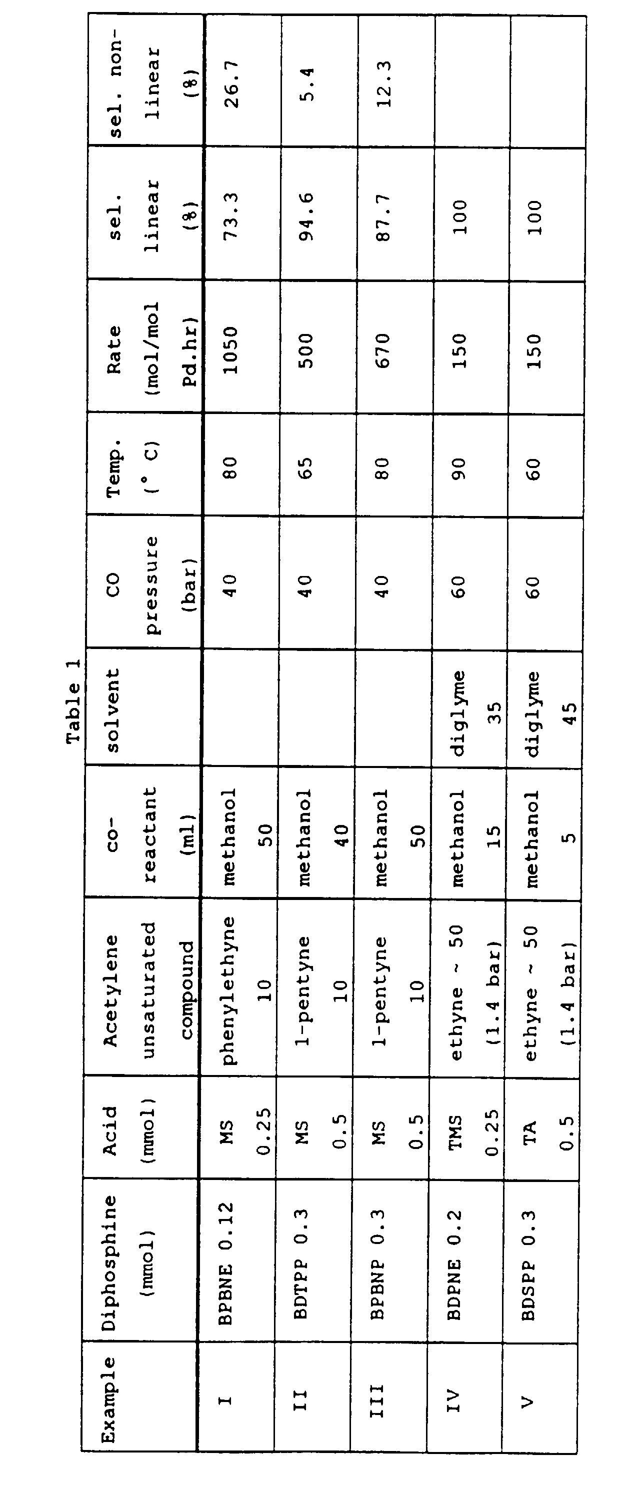

The table shows that the use of a strong acid as anion source results in the predominant formation of linear unsaturated esters

In example I the methyl ester of 2-phenylacrylic acid was formed, in Example II and III the methyl ester of 2-propylacrylic acid and in Example IV and V the methyl ester of acrylic acid. It will be appreciated that methylacrylate is also formed when a weak acid is used as anion source (Example V).

### Examples VI-X

The experiments were carried out in a similar manner as described with respect to Examples I-V, with the difference that as anion source a weak acid, or a sterically hindered acid was used.

The catalyst components, reactants, reaction conditions and results are compiled in Table 2.

The further abbreviations used therein, have the following meaning:
- BDEPP =: 1,3-bis(diethylphosphino)propane
- PPA =: phenylphosphonic acid
- ACA =: 9-anthracenylcarboxylic acid
- BDPPP =: 1,3-bis(diphenylphosphino)propane

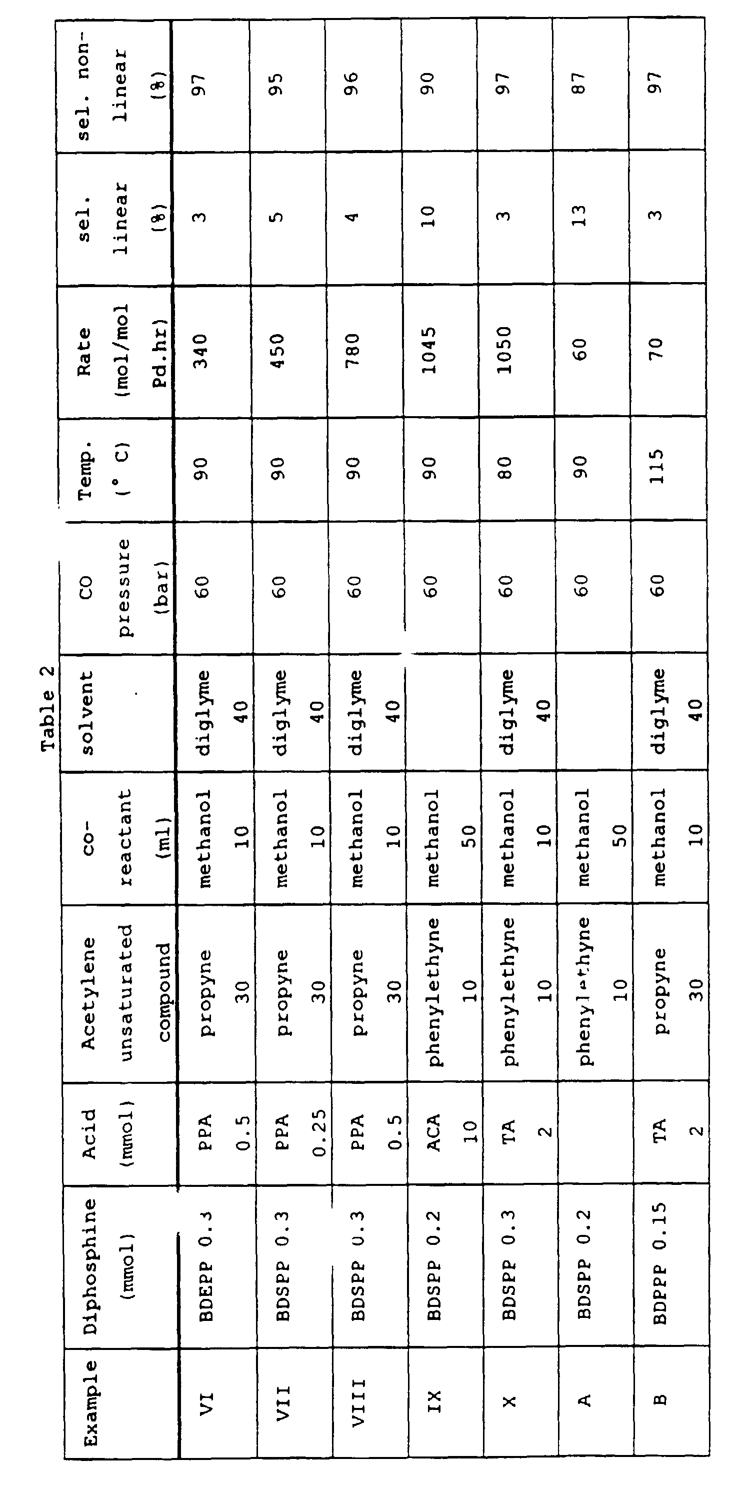

The table shows that the use of a weak acid, or a sterically hindered acid, results in the predominant formation of a non-linear unsaturated ester.

In Examples VI-VIII the main product was methylmethacrylate, in Examples IX and X the methyl ester of 1-phenylacrylic acid.

### Example A (for comparison, not according to the invention)

An experiment was carried out, substantially according to Example IX, with the difference that the catalyst composition did not contain (an acid) as anion source. The results are included in table 2, showing that the carbonylation proceeded at a much lower rate than in the experiment of Example IX.

### Example B (for comparison, not according to the invention)

An experiment was carried out, substantially according to Example VI, with the differences that the bidentate diphosphine was bis-(diphenylphosphino)propane instead of bis-(diethylphosphino)propane and that the anion source was trifluoroacetic acid instead of phenylphosphonic acid. The results are included in Table 2, showing that even at a reaction temperature of 115 °C the reaction rate was only 70 mol/mol Pd.hr.

## Claims

1. A process for the carbonylation of acetylenically unsaturated compounds with carbon monoxide in the presence of a nucleophilic compound having one or more mobile hydrogen atoms as co-reactant, and of a catalyst system based on
(a) a source of palladium cations;
(b) a bidentate diphosphine of the general formula
R¹R²P-R-PR³R⁴ (I)
wherein each of R¹,R²,R³ and R⁴ independently represents a substituted or non-substituted aliphatic or cycloaliphatic group, or R¹ together with R², and/or R³ together with R⁴ represent a substituted or non-substituted bivalent cyclic group with at least 5 ring atoms whereby the two free valencies of such a cylic group are linked to a single phosphorus atom, and R represents a bivalent organic bridging group containing from 1 to 4 atoms in the bridge, and
(c) a source of non- or weakly coordinating anions derivable from strong acids having a pKa not greater than 4, or a source of anions derivable from sterically hindered carboxylic acids.

2. A process as claimed in claim 1, wherein a catalyst system is used which, as regards (b) is based on a diphosphine of formula (I), wherein each of R¹,R²,R³ and R⁴ independently represents a substituted or non-substituted alkylgroup having from 1 to 6 carbon atoms.

3. A process as claimed in claim 1, wherein in the diphosphine of formula (I) one or more of R¹,R²,R³ and R⁴ represents a substituted or non-substituted cycloaliphatic group with 5 to 8 ringatoms.

4. A process as claimed in claim 1, wherein in the diphosphine of formula (I), R¹ together with R², and/or R³ together with R⁴ represent a cycloalkylene group having from 6 to 10 ring atoms.

5. A process as claimed in any one of claims 1-4, wherein R represents an ethylene or a trimethylene group.

6. A process as claimed in any of claims 1-5, wherein the catalyst system, as regards (b) is based on 1,3-bis(di-secondary-butyl-phosphino)propane, a 1,2-bis(9-phosphabicyclononyl)ethane, a 1,2-bis(9-phosphabicyclononyl)propane or on a 1,3-bis(9-phosphabicyclononyl)propane.

7. A process as claimed in any of claims 1-6, wherein a catalyst system is used which, as regards (c), is based on an acid having a pKa not greater than 4, or a salt of such an acid.

8. A process as claimed in any of claims 1-6, wherein a catalyst system is used which, as regards (c), is based on a sterically hindered carboxylic acid or a salt thereof.

9. A process as claimed in any of claims 1-8, wherein as starting material an acetylenically unsaturated compound having from 2 to 9 carbon atoms is used.

10. A process as claimed in claim 9, wherein ethyne, propyne or phenylethyne is used.

11. A process as claimed in any of claims 1-10, wherein as nucleophilic co-reactant a compound is applied, selected from the group consisting of alcohols, phenols and thiols.

12. A process as claimed in claim 11, wherein as nucleophilic co-reactant an alcohol having from 1 to 6 carbon atoms such as methanol or 1-butanol is used.

13. Use of a catalyst system, as defined in claim 7, in the preparation of linear carbonylation products.

14. Use of a catalyst system, as defined in claim 8, in the preparation of branched carbonylation products.

## Patentansprüche

1. Verfahren zur Carbonylierung von acetylenisch ungesättigten Verbindungen mit Kohlenmonoxid in Anwesenheit einer nukleophilen Verbindung, die ein oder mehrere bewegliche Wasserstoffatome aufweist, als Co-Reaktante und eines Katalysatorsystems auf der Basis von
(a) einer Quelle von Palladiumkationen;
(b) einem Bidentatdiphosphin mit der allgemeinen Formel
R¹R²P-R-PR³R⁴ (I),
worin jeder der Reste R¹, R², R³ und R⁴ unabhängig voneinander eine substituierte oder unsubstiuierte aliphatische oder cycloaliphatische Gruppe darstellt oder R¹ zusammen mit R², und/oder R³ zusammen mit R⁴, eine substituierte oder unsubstituierte zweiwertige cyclische Gruppe mit wenigstens 5 Ringatomen bedeuten, wobei die beiden freien Valenzen einer solchen cyclischen Gruppe an ein einziges Phosphoratom gebunden sind, und R eine zweiwertige organische Brückengruppe mit einem Gehalt an 1 bis 4 Atomen in der Brücke darstellt, und
(c) einer Quelle von nichtkoordinierenden oder schwach koordinierenden Anionen, die von starken Säuren mit einem pKa-Wert von nicht größer als 4 ableitbar sind, oder einer Quelle von Anionen, die von sterisch gehinderten Carbonsäuren ableitbar sind.

2. Verfahren nach Anspruch 1, worin ein Katalysatorsystem eingesetzt wird, das hinsichtlich (b) auf einem Diphosphin der Formel (I) beruht, worin jeder der Reste R¹, R², R³ und R⁴ unabhängig voneinander eine substituierte oder unsubstituierte Alkylgruppe mit 1 bis 6 Kohlenstoffatomen darstellt.

3. Verfahren nach Anspruch 1, worin in dem Diphosphin der Formel (I) einer oder mehrere der Reste R¹, R², R³ und R⁴ eine substituierte oder unsubstituierte cycloaliphatische Gruppe mit 5 bis 8 Ringatomen bedeutet bzw. bedeuten.

4. Verfahren nach Anspruch 1, worin in dem Diphosphin der Formel (I) R¹ zusammen mit R², und/oder R³ zusammen mit R⁴, eine Cycloakylengruppe mit 6 bis 10 Ringatomen bedeuten.

5. Verfahren nach einem der Ansprüche 1 bis 4, worin R eine Ethylen- oder eine Trimethylengruppe darstellt.

6. Verfahren nach einem der Ansprüche 1 bis 5, worin das Katalysatorsystem hinsichtlich (b) auf 1,3-Bis(disek.butylphosphino)propan, 1,2-Bis(9-phosphabicyclononyl)ethan, 1,2-Bis(9-phosphabicyclononyl)propan oder auf 1,3-Bis(9-phosphabicyclononyl)propan aufgebaut ist.

7. Verfahren nach einem der Ansprüche 1 bis 6, worin ein Katalysatorsystem verwendet wird, das hinsichtlich (c) auf einer Säure mit einem pKa-Wert von nicht größer als 4 oder auf einem Salz einer solchen Säure aufgebaut ist.

8. Verfahren nach einem der Ansprüche 1 bis 6, worin ein Katalysatorsystem verwendet wird, das hinsichtlich (c) auf einer sterisch gehinderten Carbonsäure oder einem Salz hievon aufgebaut ist.

9. Verfahren nach einem der Ansprüche 1 bis 8, worin als Ausgangsmaterial eine acetylenisch ungesättigte Verbindung mit 2 bis 9 Kohlenstoffatomen verwendet wird.

10. Verfahren nach Anspruch 9, worin Ethin, Propin oder Phenylethin verwendet wird.

11. Verfahren nach einem der Ansprüche 1 bis 10, worin als nukleophile Co-Reaktante eine Verbindung angewendet wird, die aus der aus Alkoholen, Phenolen und Thiolen bestehenden Gruppe ausgewählt ist.

12. Verfahren nach Anspruch 11, worin als nukleophile Co-Reaktante ein Alkohol mit 1 bis 6 Kohlenstoffatomen, wie Methanol oder 1-Butanol, eingesetzt wird.

13. Anwendung eines Katalysatorsystems, wie in Anspruch 7 definiert, in der Herstellung von linearen Carbonylierungsprodukten.

14. Anwendung eines Katalysatorsystems, wie in Anspruch 8 definiert, in der Herstellung von verzweigten Carbonylierungsprodukten.

## Revendications

1. Procédé de carbonylation de composés acétyléniquement insaturés avec le monoxyde de carbone en présence d'un composé nucléophile possédant un ou plusieurs atomes d'hydrogène mobiles à titre de coréactif, et un système catalytique à base
(a) d'une source de cations palladium;
(b) d'une diphosphine bidentate de la formule générale :
R¹R²P-R-PR³R⁴ (I)
dans laquelle chacun des symboles R¹, R², R³ et R⁴ représente indépendamment un groupe cycloaliphatique ou aliphatique, substitué ou non substitué, ou bien R¹ ensemble avec R² et/ou R³ ensemble avec R⁴ représentent un radical cyclique bivalent, substitué ou non substitué, comportant au moins 5 atomes cycliques, où les deux valences libres d'un tel radical cyclique sont liées à un seul atome de phosphore et R représente un radical de pontage organique bivalent contenant de 1 à 4 atomes dans le pont, et
(c) d'une source d'anions non coordinants ou faiblement coordinants, provenant d'acides forts possédant un pKa non supérieur à 4, ou une source d'anions provenant d'acides carboxyliques stériquement empêchés.

2. Procédé suivant la revendication 1, caractérisé en ce que l'on utilise un système catalytique qui, en ce qui concerne (b), est basé sur une diphosphine de la formule (I), dans laquelle chacun des symboles R¹, R², R³ et R⁴ représente indépendamment un radical alkyle substitué ou non substitué, qui comporte de 1 à 6 atomes de carbone.

3. Procédé suivant la revendication 1, caractérisé en ce que, dans la diphosphine de la formule (I), un ou plusieurs des symboles R¹, R², R³ et R⁴ représentent un groupe cycloaliphatique à 5 à 8 atomes cycliques, substitué ou non substitué.

4. Procédé suivant la revendication 1, caractérisé en ce que, dans la diphosphine de la formule (I), R¹ ensemble avec R² et/ou R³ ensemble avec R⁴ représentent un groupe cycloalkylène possédant de 6 à 10 atomes cycliques.

5. Procédé suivant l'une quelconque des revendications 1 à 4, caractérisé en ce que R représente le radical éthylène ou le radical triméthylène.

6. Procédé suivant l'une quelconque des revendications 1 à 5, caractérisé en ce que le système catalytique, en ce qui concerne (b) est basé sur le 1,3-bis-[dibutyl(secondaire)phosphino]-propane, le 1,2-bis-(9-phosphabicyclononyl)-éthane, le 1,2-bis-(9-phosphabicyclononyl)-propane ou le 1,3-bis-(9-phosphabicyclononyl)-propane.

7. Procédé suivant l'une quelconque des revendications 1 à 6, caractérisé en ce que l'on utilise un système catalytique qui, en ce qui concerne (c), est basé sur un acide possédant un pKa non supérieur à 4, ou un sel d'un tel acide.

8. Procédé suivant l'une quelconque des revendications 1 à 6, caractérisé en ce que l'on utilise un système catalytique qui, en ce qui concerne (c), est basé sur un acide carboxylique stériquement empêché ou un sel de celui-ci.

9. Procédé suivant l'une quelconque des revendications 1 à 8, caractérisé en ce que, à titre de matière de départ, on utilise un composé acétyléniquement insaturé possédant de 2 à 9 atomes de carbone.

10. Procédé suivant la revendication 9, caractérisé en ce que l'on utilise de l'éthyne, du propyne ou du phényléthyne.

11. Procédé suivant l'une quelconque des revendications 1 à 10, caractérisé en ce qu'à titre de coréactif nucléophile, on applique un composé choisi dans le groupe formé par les alcools, les phénols et les thiols.

12. Procédé suivant la revendication 11, caractérisé en ce que, à titre de coréactif nucléophile, on utilise un alcool possédant de 1 à 6 atomes de carbone, comme le méthanol ou le 1-butanol.

13. Utilisation d'un système catalytique suivant la revendication 7, pour la préparation de produits de carbonylation linéaires.

14. Utilisation d'un système catalytique, tel que défini dans la revendication 8, pour la préparation de produits de carbonylation ramifiés.
